# EUROPÄISCHE PATENTSCHRIFT

(11) **EP 0 815 112 B1**
(45) Veröffentlichungstag und Bekanntmachung des Hinweises auf die Patenterteilung: **08.08.2001**
(21) Anmeldenummer: 96907470.7
(22) Anmeldetag: 14.03.1996
(51) Int. Cl.: C07D 491/06, A61K 31/55

(54) **VERFAHREN ZUR ISOLIERUNG VON GALANTHAMIN**
PROCESS FOR ISOLATING GALANTHAMINE
PROCEDE D'ISOLATION DE GALANTHAMINE

(30) Priorität: 17.03.1995 DE 19509663
(43) Veröffentlichungstag der Anmeldung: 07.01.1998
(60) Teilanmeldung: 01100944.6
(73) Patentinhaber: LTS Lohmann Therapie-Systeme AG, 56626 Andernach (DE)
(72) Erfinder: HILLE, Thomas, D-56564 Neuwied (DE); HOFFMANN, Hans-Rainer, D-56564 Neuwied (DE); KREH, Mirko, D-35039 Marburg (DE); MATUSCH, Rudolf, D-35041 Marburg (DE)
(74) Vertreter: Flaccus, Rolf-Dieter, Dr.
(86) Internationale Anmeldenummer: EP9601094
(87) Internationale Veröffentlichungsnummer: WO9629332

(56) Entgegenhaltungen:
- DE-B- 1 193 061
- PHARMAZIE, Bd. 38, Nr. 9, 1983, BERLIN DE, Seiten 596-600, XP000570492 R. K. MÜLLER: "Parameter für die flüssig/flüssig-Extraktion toxikologisch relevanter organischer Verbindungen"
- CHEMICAL ABSTRACTS, vol. 82, no. 10, 10.März 1975 Columbus, Ohio, US; abstract no. 64386f, V. G. KRAMARENKO : "Effect of PH of the medium on the extraction of alkaloids with organic solvents" Seite 463; XP002003767 & FARMATSIYA, Bd. 23, Nr. 5, 1975, Seiten 29-32,
- CHEMICAL ABSTRACTS, vol. 66, no. 20, 15.Mai 1967 Columbus, Ohio, US; abstract no. 88625d, V. V. MIKHNO : "The comparative appraisal of methods for the isolation of galanthamine and securinine from biological material" Seite 8310; XP002003768 & FARM. ZH., Bd. 21, Nr. 6, 1966, Seiten 28-29,
- PHYTOCHEMISTRY, Bd. 26, Nr. 5, 1987, Seiten 1519-1524, XP000571959 J. BASTIDA ET AL: "Alkaloids from Narcissus confusus"

## Beschreibung

Gegenstand der vorliegenden Erfindung ist ein Verfahren zur Isolierung des Alkaloids Galanthamin, das nach diesem Verfahren hergestellte Galanthamin selbst, die Verwendung des so hergestellten Galanthamins in galenischen Zubereitungsformen, sowie das so hergestellte Galanthamin zur Behandlung des Engwinkelglaukoms, der Alzheimerschen Krankheit und der Alkohol- und Nikotinabhängigkeit.

Galanthamin (4a,5,9,10,11,12-Hexahydro-3-methoxy-11-methyl-6-H-benzofuro-(3a,3,2-ef)-(2)-benzazepin-6-ol) ist ein tetracyclisches Alkaloid, das aufgrund seiner pharmakologischen Eigenschaften zur Gruppe der reversibel wirkenden Cholinesterasehemmstoffen gehört und in seinen Wirkungen dem Physostigmin und dem Neostigmin nahesteht. Es besitzt jedoch auch eigene spezifische Eigenschaften, wie beispielsweise mit Morphin vergleichbare stark analgetische Wirkungen. Als Cholinesterasehemmer besitzt Galanthamin aufgrund seiner im Vergleich zu Physostigmin und Neostigmin geringeren Toxizität eine drei- bis sechsmal größere therapeutische Breite. Dieser Vorteil wiegt seine dosisbezogene etwas geringere Cholinesterasehemmwirkung auf. Galanthamin wird bei Poliomyelitis und verschiedenen Erkrankungen des Nervensystems eingesetzt, hauptsächlich jedoch bei der Behandlung des Engwinkelglaukoms und als Antidot nach Curare Applikationen. Versuchsweise wird Galanthamin bei der Alzheimerschen Krankheit eingesetzt. In jüngster Zeit wurde auch die Behandlung der Alkohol- und Nikotinabhängigkeit beschrieben (DE-OS 40 10 079, DE-OS 43 01 782).

Sowohl die Therapie der Alzheimerschen Krankheit, der Alkohol- und Nikotinabhängigkeit als auch des Engwinkelglaukoms erfordern langwirksame, den besonderen Umständen angepasste Arzneiformen. Als solche bietet sich bei der Behandlung des Engwinkelglaukoms eine Augensalbe an. Schwierige Therapieschemata und Dauerinfusionen kommen aus naheliegenden Gründen zur Behandlung der Alzheimerschen Krankheit, der Alkohol- oder Nikotinabhängigkeit nicht in Frage. Bei diesen Erkrankungen bietet sich vielmehr ein transdermales therapeutisches System (TTS) an, wie es beispielsweise in der DE-OS 43 01 783 beschrieben wird. Weder die intakte Haut noch die Cornea des Auges gestatten eine Resorption von Wirkstoffsalzen. Galanthaminhydrochlorid oder Galanthaminhydrobromid können daher bei der Therapie des Engwinkelglaukoms, der Alzheimerschen Krankheit oder der Alkohol- oder Nikotinabhängigkeit mit Salben oder dem TTS nicht verwendet werden. Aus diesem Grund muß also die reine Galanthaminbase eingesetzt werden.

Aufgrund seiner komplizierten tetracyclischen Struktur mit drei optisch aktiven Kohlenstoffatomen ist eine wirtschaftliche Synthese der Galanthaminbase nicht möglich. Galanthamin wird daher üblicherweise aus Pflanzen der Amaryllidaceen, beispielsweise aus Galanthusarten, wie dem Schneeglöckchen oder Leucojum aestivum, isoliert. Diese Pflanzen haben zwar den Vorteil, Galanthamin in Konzentrationen von bis zu 0,3 %, bei gleichzeitig geringem Anteil an Nebenalkaloiden zu enthalten, so daß das in der DE-PS 11 93 061 beschriebene Extraktionsverfahren Anwendung finden kann. Sowohl die Galanthusarten als auch Leucojum aestivum stehen jedoch unter Naturschutz. Zum anderen verwendet das in der DE-PS 11 93 061 beschriebene Extraktionsverfahren vorzugsweise chlorierte Kohlenwasserstoffe, die aus toxikologischen Gründen in Verruf geraten sind. Die Arzneibücher der westlichen Welt fordern daher den Restgehalt an chlorierten Kohlenwasserstoffen auf < 10 ppm zu beschränken. Der Einsatz chlorierter Kohlenwasserstoffe sollte folglich bei der Bereitstellung von Arzneistoffen möglichst vermieden werden. Darüber hinaus muß bei dem bekannten Verfahren der Lösungsmittelextrakt an Aluminiumoxyd adsorbiert werden, um die Abtrennung der Harzstoffe und Nebenalkaloide zu gewährleisten. Aus der nach dem Abfiltieren des Aluminiumoxyds erhaltenen Lösung wird das Galanthamin dann über das Galanthaminhydrobromid gereinigt, mit der damit verbundenen Entsorgung von Halogensalzen. Für den Einsatz in Salben und dem TTS muß dann die Galanthaminbase auch noch aus diesem Galanthaminhydrobromid freigesetzt werden.

Aufgabe der vorliegenden Erfindung ist daher die Bereitstellung eines Verfahrens zur Isolierung und Reinigung von Galanthamin, das die Nachteile der im Stand der Technik bekannten Verfahren nicht aufweist. Insbesondere soll die Reinigung erleichtert werden, der Einsatz von chlorierten Kohlenwasserstoffen und die Reinigung über Galanthaminsalze vermieden werden. Die Aufgabe wird erfindungsgemäß durch ein Verfahren mit den Kennzeichen des Anspruchs 1 gelöst. Vorzugsweise Ausgestaltungen sind in den Unteransprüchen charakterisiert.

Gegenstand der Erfindung im einzelnen ist ein Verfahren zur Isolierung von Galanthamin aus biologischem Material, das aus landwirtschaftlich angebauten Amaryllidaceenarten oder aus solchen, die allgemein als "Unkraut" gelten und nicht unter Naturschutz stehen, vorzugsweise aus den Zwiebeln dieser Pflanzen, gewonnen wird. Zu diesen Amaryllidaceen zählen beispielsweise Narzissen bzw. Crinumarten. Besonders geeignet sind Narcissus pseudonarcissus "Carlton" oder die asiatische Kletterpflanze Crinum amabile. Obwohl diese Pflanzen nur etwa ein Zehntel der Galanthaminmenge der unter Naturschutz stehenden Pflanzen und darüber hinaus auch noch bis zu zwölf Nebenalkaloide enthalten, gelingt es überraschenderweise mit dem erfindungsgemäßen Verfahren hieraus die Galanthaminbase in für Arzneimittel geeigneter Reinheit zu isolieren.

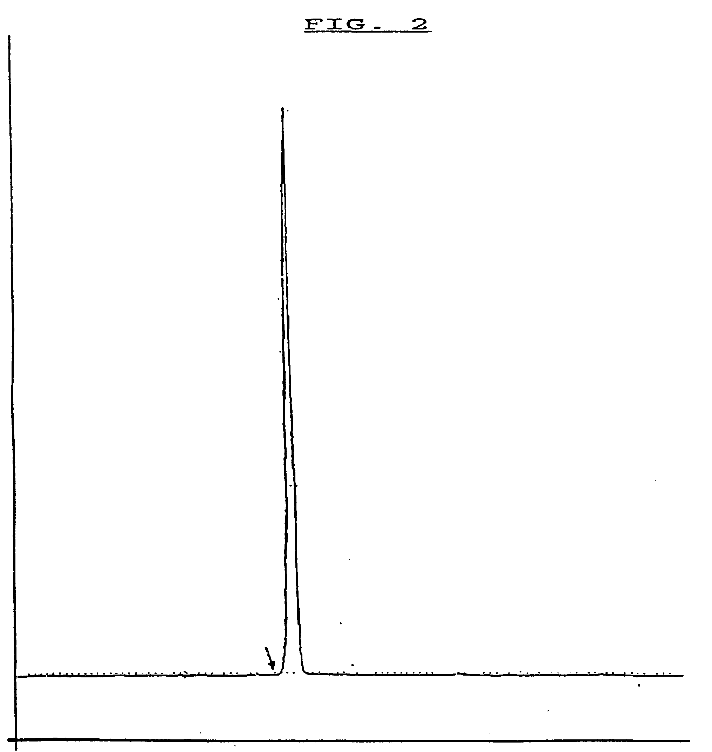

Erfindungsgemäß wird Galanthamin enthaltendes, biologisches Material, das vorzugsweise zerkleinert und mit Alkalipulver, vorzugsweise mit Natriumhydroxid-Plätzchen, Soda, Pottasche oder ähnlichen Salzen, die geeignet sind, Basen aus biologischem Material freizusetzen und zur Darstellung pharmokologischer Wirkstoffe geeignet sind, vermischt wird, mit toxikologisch unbedenklichen organischen Lösungsmitteln extrahiert und das Galanthamin aus diesem Extrakt durch Flüssig/Flüssig Extraktion gereinigt. Von besonderer Bedeutung für den Erfolg des erfindungsgemäßen Verfahrens ist die Einhaltung des pH-Wertes in der ersten Stufe der Flüssig/Flüssig-Extraktion. Die Flüssig/Flüssig Extraktion wird in einer ersten Stufe bei einem pH-Wert von etwa 4 und in einer zweiten Stufe bei einem pH-Wert von etwa 9 durchgeführt. Zur Einstellung des pH-Wertes kann nebem konzentriertem Ammoniak auch Soda-Lösung oder eine andere Base eingesetzt werden. Als toxikologisch unbedenkliches organisches Lösungsmittel wird Diethylether oder Spezialbenzin eingesetzt. Vorzugsweise wird Spezialbenzin verwendet, da sich damit bereits eine gewisse Vorreinigung erzielen läßt. Das Lösungsmittel wird entfernt, das Galanthamin aus einem geeigneten Lösungsmittel, vorzugsweise aus Isopropanol umkristallisiert. Man erhält die weiße Galanthaminbase mit einem Schmelzpunkt von 129 bis 130°C. Die Reinheit des so erhaltenen Galanthamins zeigt sich im HPLC-Chromatogramm (Fig.2).

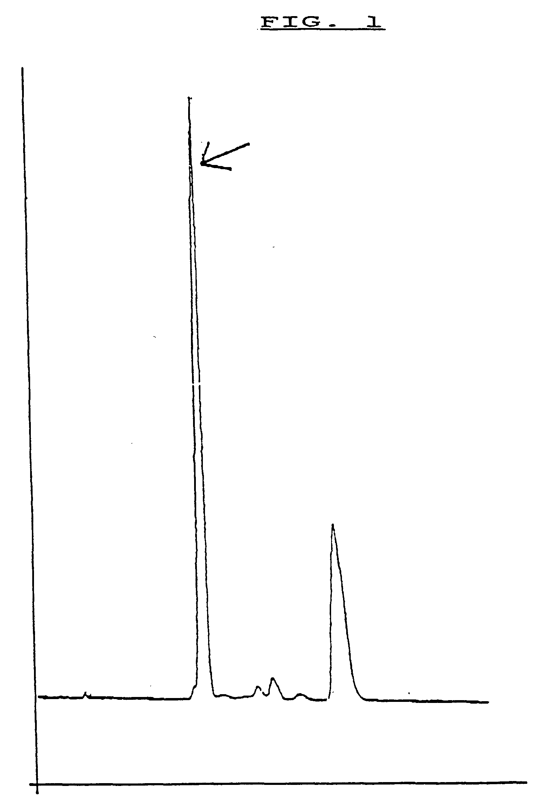

Dieses Ergebnis verblüfft umso mehr, als sich das bisher verwendete Verfahren aus der DE-PS 11 93 061 zur Isolierung des Galanthamins aus biologischem Material, das wenig Galanthamin und bis zu 12 Nebenalkaloide enthält, als ungeeignet herausstellte. Mit dem in der DE-PS 11 93 061 beschriebenen Verfahren entsteht eine nicht zu brechende Emulsion, so daß eine Extraktion nicht möglich ist. Ein leicht modifiziertes Verfahren führte zu einem öligen Rückstand, der laut HPLC-Chromatogramm durch mindestens vier Substanzen verunreinigt ist (Fig.1).

Es muß daher als ausgesprochen überraschend angesehen werden, daß sich aus biologischem Material, das wenig Galanthamin neben einer großen Anzahl an Nebenalkaloiden enthält, mit Hilfe des einfachen erfindungsgemäßen Verfahrens die freie Galanthaminbase in reiner Form und guter Ausbeute isolieren läßt, ohne daß ein zusätzlicher Adsorptionsschritt an Aluminiumoxyd erforderlich ist.

Die erfindungsgemäß hergestellte Galanthaminbase ist zur Behandlung des Engwinkelglaukoms, zur Behandlung der Alzheimerschen Krankheit oder der Alkohol- und Nikotinabhängigkeit einsetzbar. Die erfindungsgemäß hergestellte Galanthaminbase kann darüber hinaus in galenischen Zubereitungsformen, wie beispielsweise in Augensalben oder in transdermalen therapeutischen Systemen verwendet werden, die ebenfalls zur Behandlung der obengenannten Krankheiten eingesetzt werden können.

Die nachfolgenden Beispiele sollen die Erfindung erläutern, ohne sie einzuschränken:

### Beispiel 1 (Vergleich) :

Analog zum Verfahren, das in der DE-PS 11 93 061 beschrieben ist, werden 10 kg luftgetrocknete, zerkleinerte Zwiebeln von Narcissus pseudonarcissus "Carlton" sorgfältig mit 2,5 l einer 8%igen wäßrigen Ammoniaklösung vermischt. Das Material quillt auf; der gesamte Ansatz verkleistert. Die zur Extraktion vorgesehene Zugabe von 23 l Dichlorethan führt zu einer Emulsion, die nicht gebrochen werden kann.

### Beispiel 2 (Vergleich):

Das im Stand der Technik bekannte Verfahren zur Isolierung des Galanthamins (DE-PS 11 93 061) wurde modifiziert. 10 kg luftgetrocknete, zerkleinerte Zwiebeln von Narcissus pseudonarcissus "Carlton" werden sorgfältig mit 400 g Natriumcarbonat vermischt. Danach werden 23 l Dichlorethan zugegeben. Das Gemisch bleibt 10 Stunden stehen; anschließend wird das Lösungsmittel abgegossen. Die Zwiebeln werden erneut mit 23 l Dichlorethan übergossen, das nach 2 bis 3 Stunden abgegossen wird. Im Anschluß daran werden die Zwiebeln ein drittes Mal mit 17 l Dichlorethan versetzt, das jedoch direkt wieder abgegossen wird. Die vereinigten Dichlorethanextrakte werden mittels 10%iger Schwefelsäure extrahiert (2 x je 600 ml; 2 x je 300 ml). Die sauren Extrakte werden vereinigt und durch Ausschütteln mit Diethylether von den Spuren des Dichlorethans gereinigt. Danach werden unter Rühren und Kühlung auf 15 bis 20°C etwa 200 ml einer 25%igen wäßrigen Ammoniaklösung bis zur alkalischen Lackmusreaktion zugefügt. Der pH-Wert liegt bei 7 bis 8. Anders als im Stand der Technik angegeben fallen die Nebenalkaloide nicht aus. Die alkalische Lösung wird mit Kochsalz gesättigt und mit Diethylether extrahiert. Nach Abdampfen des Ethers bleibt, anders als im Stand der Technik angegeben, ein vernachlässigbar kleiner Rückstand übrig. Durch Sättigung mit Pottasche wird der pH-Wert der wäßrigen Phase auf etwa 14 eingestellt. Die wäßrige Phase wird mehrfach mit Diethylether extrahiert. Die vereinigten Etherextrakte werden zur Trockne eingedampft, der zurückbleibende galanthaminhaltige Rückstand in Aceton (50 ml) gelöst. Anders als im Stand der Technik angegeben, bildet sich kein Niederschlag. Man ergänzt 350 ml Aceton, fügt 200 g Aluminiumoxid hinzu und rührt 45 Minuten lang. Das Aluminiumoxid wird abfiltriert und zweimal mit je 100 ml Aceton gewaschen. Die vereinigten Acetonlösungen werden zur Trockne eingedampft. Man erhält 1,3 g eines öligen Rückstandes, der mit Hilfe der HPLC untersucht wird. Das Chromatogramm ist in Figur 1 dargestellt. Der Hauptpeak, das Galanthamin , ist gekennzeichnet. Man erkennt deutlich, daß das auf diese Weise isolierte Galanthamin durch mindestens vier Substanzen verunreinigt ist.

### Beispiel 3:

100 kg luftgetrocknete, zerkleinerte Zwiebeln von Narcissus pseudonarcissus "Carlton" werden mit 4 kg Natriumcarbonat sorgfältig vermischt. Die Mischung wird in drei gleiche Teile geteilt und mit je 15 l Spezialbenzin 80/110 übergossen. Man läßt 24 Stunden stehen. Die Lösungsmittel werden je zweimal erneuert, gesammelt und im schwachen Vakuum zur Trockne eingeengt. Die Extrakte werden in 2%iger wäßriger Schwefelsäure aufgenommen und mit konzentrierter wäßriger Ammoniaklösung auf einen pH-Wert von 4 eingestellt. Anschließend wird fünfmal mit Diethylether extrahiert. Die wäßrige Phase wird mit konzentriertem Ammoniak auf einen pH-Wert von 9 eingestellt und fünfmal mit Diethylether extrahiert. Diese Etherfraktionen werden gesammelt, mit Natriumsulfat getrocknet und eingeengt. Es werden 20 g eines schwach gelb gefärbten öligen Rückstandes erhalten, der aus heißem Isopropanol umkristallisiert wird. Man erhält 10 g weiße Galanthaminbase mit einem Schmelzpunkt von 129 - 130°C. Im HPLC-Chromatogramm ist ausschließlich ein Peak zu erkennen (Fig.2).

## Patentansprüche

1. Verfahren zur Isolierung von Galanthamin, dadurch gekennzeichnet, daß Galanthamin enthaltendes, biologisches Material mit toxikologisch unbedenklichen organischen Lösungsmitteln extrahiert wird und das Galanthamin aus diesem Extrakt durch Flüssig/Flüssig Extraktion gereinigt wird, wobei die Flüssig/Flüssig Extraktion in der ersten Stufe bei einem pH-Wert von etwa 4 durchgeführt wird.

2. Verfahren gemäß Anspruch 1, dadurch gekennzeichnet, daß das biologische Material vor der Extraktion zerkleinert und mit Alkalipulver vermischt wird.

3. Verfahren gemäß einem der Ansprüche 1 oder 2, dadurch gekennzeichnet, daß die Flüssig/Flüssig Extraktion in der zweiten Stufe bei einem pH-Wert von etwa 9 durchgeführt wird.

4. Verfahren gemäß einem der Ansprüche 1 bis 3, dadurch gekennzeichnet, daß als Alkalipulver Natriumcarbonat eingesetzt wird.

5. Verfahren gemäß einem der Ansprüche 1 bis 4, dadurch gekennzeichnet, daß als toxikologisch unbedenkliches organisches Lösungsmittel Diethylether oder Spezialbenzin, vorzugsweise Spezialbenzin eingesetzt wird.

6. Verfahren gemäß einem der Ansprüche 1 bis 5, dadurch gekennzeichnet, daß das erhaltene Galanthamin aus einem geeigneten Lösungsmittel umkristallisiert wird.

7. Verfahren gemäß Anspruch 5, dadurch gekennzeichnet, daß als Lösungsmittel Isopropanol eingesetzt wird.

8. Verfahren gemäß einem der Ansprüche 1 bis 7, dadurch gekennzeichnet, daß als biologisches Material Pflanzenteile der Amaryllidaceen verwendet wird.

9. Verfahren nach Anspruch 8, dadurch gekennzeichnet, daß als Pflanzenteile Zwiebeln verwendet werden.

10. Verfahren gemäß einem der Ansprüche 1 bis 9, dadurch gekennzeichnet, daß als Amaryllidaceen Narzissenarten oder Crinumarten verwendet werden.

11. Verfahren nach Anspruch 10, dadurch gekennzeichnet, daß als Narzissenart Narcissus pseudonarcissus "Carlton" beziehungsweise als Crinumart Crinum amabile verwendet werden.

12. Verfahren gemäß einem der Ansprüche 1 bis 11, dadurch gekennzeichnet, daß Galanthamin in einer Reinheit von ≥ 99 % erhalten wird.

13. Galanthamin in pharmazeutischer Qualität mit ≥ 99%iger Reinheit, hergestellt nach einem Verfahren gemäß einem der Ansprüche 1 bis 12.

14. Galenische Zubereitungsformen, gekennzeichnet durch einen Gehalt an Galanthamin nach Anspruch 13.

15. Verwendung des Galanthamins nach Anspruch 13 zur Herstellung von Augensalben.

16. Verwendung des Galanthamins nach Anspruch 13 zur Herstellung von transdermalen therapeutischen Systemen.

17. Verwendung von Galanthamin nach Anspruch 13 zur Herstellung eines Mittels zur Behandlung des Engwinkelglaukoms.

18. Verwendung von Galanthamin nach Anspruch 13 zur Herstellung eines Mittels zur Behandlung der Alzheimerschen Krankheit.

19. Verwendung von Galanthamin nach Anspruch 13 zur Herstellung eines Mittels zur Behandlung der Alkoholabhänigkeit.

20. Verwendung von Galanthamin nach Anspruch 13 zur Herstellung eines Mittels zur Behandlung der Nikotinabhängigkeit.

21. Pharmazeutisches Präparat, dadurch gekennzeichnet, daß es neben den an sich bekannten pharmazeutischen Hilfsstoffen Galanthamin gemäß Anspruch 13 enthält.

22. Pharmazeutisches Präparat gemäß Anspruch 21 zur topischen Applikation.

23. Pharmazeutisches Präparat gemäß Anspruch 21 oder 22 zur Behandlung des Engwinkelglaukoms.

24. Pharmazeutisches Präparat gemäß Anspruch 21 oder 22 zur Behandlung der Alzheimerschen Krankheit.

25. Pharmazeutisches Präparat gemäß Anspruch 21 oder 22 zur Behandlung der Alkoholabhängigkeit.

26. Pharmazeutisches Präparat gemäß Anspruch 21 oder 22 zur Behandlung der Nikotinabhängigkeit.

## Claims

1. A process for the isolation of galanthamine characterized in that gaianthamine-containing, biological material is extracted with toxicologically safe organic solvents and that the galanthamine from this extract is purified by means of liquid-liquid extraction, said liquid-liquid extraction being carried out in the first stage at a pH-value of about 4.

2. The process according to claim 1 characterized in that the biological material is comminuted and mixed with alkali powder prior to the extraction.

3. The process according to any one of claims 1 or 2 characterized in that the liquid-liquid extraction in carried out in the second stage at a pH-value of about 9.

4. The process according to any one of claims 1 to 3 characterized in that sodium carbonate is used as alkali powder.

5. The process according to any one of claims 1 to 4 characterized in that diethyl ether or special boiling-point gasoline is used as toxicologically safe organic solvent, special boiling-point gasoline being preferred.

6. The process according to any one of claims 1 to 5 characterized in that the obtained galanthamine is recrystallized from a suitable solvent.

7. The process according to claim 6 characterized in that isopropanol is used as solvent.

8. The process according to any one of claims 1 to 7 characterized in that plant parts of amaryllidaceae, are used as biological material.

9. The process according to claim 8 characterized in that bulbs are used as plant parts.

10. The process according to any one of claims 1 to 9 characterized in that narcissi species or crinum species are used as amaryllidaceae.

11. The process according to claim 10 characterized in that as narcissi species narcicissus pseudonarcissus "Carlton", or as crinum species Crinum amabile are used.

12. Process according to one of Claims 1 to 11, characterized in that galanthamine is obtained in a purity of ≥ 99%.

13. Galanthamine in pharmaceutical quality with a purity of ≥99 %, produced according to a process in accordance with any one of claims 1 to 12.

14. Galenic administration forms characterized by a content of galanthamine in accordance with claim 13.

15. The use of the galanthamine according to claim 13 for the production of ophthalmic ointments.

16. The use of the galanthamine according to claim 13 for the production of transdermal therapeutic systems.

17. The use of the galanthamine according to claim 13 for the production of a drug for the treatment of narrow-angle glaucoma.

18. The use of the galanthamine according to claim 13 for the production of a drug for the treatment of Alzheimer's disease.

19. The use of the galanthamine according to claim 13 for the production of a drug for the treatment of alcohol dependence.

20. The use of the galanthamine according to claim 13 for the production of a drug for the treatment of nicotine dependence.

21. A pharmaceutical preparation characterized in that it comprises galanthamine according to claim 13 in addition to pharmaceutical adjuvants known per se.

22. The pharmaceutical preparation according to claim 21 for topical application.

23. The pharmaceutical preparation according to claim 21 or 22 for the treatment of narrow-angle glaucoma.

24. The pharmaceutical preparation according to claim 21 or 22 for the treatment of Alzheimer's disease.

25. The pharmaceutical preparation according to claim 21 or 22 for the treatment of alcohol dependence.

26. The pharmaceutical preparation according to claim 21 or 22 for the treatment of nicotine dependence.

## Revendications

1. Procédé pour l'isolation de galanthamine, caractérisé en ce qu'on extrait une matière biologique contenant de la galanthamine avec des solvants organiques acceptables du point de vue toxicologique et on purifie la galanthamine à partir de cet extrait par extraction de type liquide/liquide, dans lequel on effectue l'extraction de type liquide/liquide, dans la première étape, à une valeur de pH d'environ 4.

2. Procédé selon la revendication 1, caractérisé en ce que la matière biologique est broyée avant l'extraction et est mélangée avec de la poudre alcaline.

3. Procédé selon l'une quelconque des revendications 1 ou 2, caractérisé en ce qu'on effectue l'extraction de type liquide/liquide, dans la deuxième étape, à une valeur de pH d'environ 9.

4. Procédé selon l'une quelconque des revendications 1 à 3, caractérisé en ce qu'on met en oeuvre, à titre de poudre alcaline, du carbonate de sodium.

5. Procédé selon l'une quelconque des revendications 1 à 4, caractérisé en ce qu'on met en oeuvre, à titre de solvant organique acceptable du point de vue toxicologique, de l'éther diéthylique ou des essences spéciales, de préférence des essences spéciales.

6. Procédé selon l'une quelconque des revendications 1 à 5, caractérisé en ce qu'on soumet la galanthamine obtenue à une recristallisation dans un solvant approprié.

7. Procédé selon la revendication 5, caractérisé en ce qu'on met en oeuvre, à titre de solvant, de l'isopropanol.

8. Procédé selon l'une quelconque des revendications 1 à 7, caractérisé en ce qu'on utilise, à titre de matière biologique, des parties de plantes des amaryllidacées.

9. Procédé selon la revendication 8, caractérisé en ce qu'on utilise, à titre de parties de plantes, des bulbes.

10. Procédé selon l'une quelconque des revendications 1 à 9, caractérisé en ce qu'on utilise, à titre d'amaryllidacées, les espèces narcisses ou les espèces Crinum.

11. Procédé selon la revendication 10, caractérisé en ce qu'on utilise, à titre d'espèce narcisse, Narcissus pseudonarcissus "Carlton", respectivement à titre d'espèce Crinum, Crinum amabile.

12. Procédé selon l'une quelconque des revendications 1 à 11, caractérisé en ce qu'on obtient de la galanthamine avec une pureté ≥ 99 %.

13. Galanthamine de qualité pharmaceutique possédant une pureté ≥ 99 %, préparée conformément à un procédé selon l'une quelconque des revendications 1 à 12.

14. Formes de préparations galéniques caractérisées par une teneur en galanthamine selon la revendication 13.

15. Utilisation de la galanthamine selon la revendication 13 pour la préparation de pommades ophtalmiques.

16. Utilisation de la galanthamine selon la revendication 13 pour la préparation de systèmes thérapeutiques transdermiques.

17. Utilisation de la galanthamine selon la revendication 13 pour la préparation d'un agent pour le traitement du glaucome à angle étroit.

18. Utilisation de la galanthamine selon la revendication 13 pour la préparation d'un agent pour le traitement de la maladie d'Alzheimer.

19. Utilisation de la galanthamine selon la revendication 13 pour la préparation d'un agent pour le traitement de la dépendance à l'alcool.

20. Utilisation de la galanthamine selon la revendication 13 pour la préparation d'un agent pour le traitement de la dépendance à la nicotine.

21. Préparation pharmaceutique caractérisé en ce qu'elle contient, outre les adjuvants pharmaceutiques connus en soi, de la galanthamine selon la revendication 13.

22. Préparation pharmaceutique selon la revendication 21 pour l'application par voie locale.

23. Préparation pharmaceutique selon la revendication 21 ou 22 pour le traitement du glaucome à angle étroit.

24. Préparation pharmaceutique selon la revendication 21 ou 22 pour le traitement de la maladie d'Alzheimer.

25. Préparation pharmaceutique selon la revendication 21 ou 22 pour le traitement de la dépendance à l'alcool.

26. Préparation pharmaceutique selon la revendication 21 ou 22 pour le traitement de la dépendance à la nicotine.
